**Europäisches Patentamt**

**European Patent Office**

**Office européen des brevets**

(19)

(11) Numéro de publication: **0 209 710 B1**

# (12) FASCICULE DE BREVET EUROPEEN

(45) Date de publication du fascicule du brevet:
**25.07.90**

(21) Numéro de dépôt: **86108012.5**

(22) Date de dépôt: **12.06.86**

(51) Int. Cl.⁵: **A61M 25/00**

(54) **Circuit fermé pour cathéter.**

(30) Priorité: **26.07.85 CH 3262/85**

(43) Date de publication de la demande:
**28.01.87 Bulletin 87/5**

(45) Mention de la délivrance du brevet:
**25.07.90 Bulletin 90/30**

(84) Etats contractants désignés:
**AT BE CH DE FR GB IT LI LU NL SE**

(56) Documents cités:
**US-A- 3 050 066**
**US-A- 3 053 257**
**US-A- 3 131 694**
**US-A- 3 211 150**
**US-A- 3 211 151**
**US-A- 3 675 658**
**US-A- 3 769 960**
**US-A- 3 818 903**
**US-A- 3 854 484**
**US-A- 4 119 101**
**US-A- 4 182 344**

(73) Titulaire: **SARCEM SA, 27 rue Cardinal-Journet Case Postale 371, CH-1217 Meyrin 1(CH)**

(72) Inventeur: **Bonello, Philippe, Chemin des Pommiers 26, CH-1218 Grand-Saconnex(CH)**
Inventeur: **Jeanmonod, Maurice, Avenue de Vaudagne 47, CH-1217 Meyrin(CH)**

(74) Mandataire: **Micheli, Michel-Pierre et al, MICHELI & CIE 118, Rue du Rhône Case Postale 47, CH-1211 Genève 6(CH)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un cathéter présentant un circuit fermé pour le remplissage d'une sorte de vessie gonflable au moyen d'un fluide, en l'occurence un liquide. Les applications principales de ce cathéter pouvant se situer dans le cadre des maladies cardio-vasculaires au niveau des vaisseaux coronaires. Les documents US-A-3.053.257 et US-A-3050.066 décrivent des cathéters de drainage de liquides hors de cavités corporelles comportant une enveloppe étanche formant aux deux extrémités du cathéter une vessie destinée à recevoir un liquide. Ce liquide peut être transféré de la vessie arrière à celle proche de l'extrémité distale du cathéter par une poussée manuelle.

Le remplissage des vessies est problématique de même que le maintien de la vessie distale dans une condition de gonflement déterminée.

Le document US-A-4.119.101 décrit un cathéter endotrachéal comprenant une vessie distale reliée à un ballon externe situé dans un container. Le ballon est relié à une source de gaz destinée à remplir la vessie et le container est relié à une source de gaz provoquant des modifications de volume du ballon.

Ces cathéters de drainage ou endotrachéal sont inutilisables en angioplastie et surtout en angioplastie coronarienne déjà du fait de leur grande dimension.

Le document US-A-3.769.960 décrit un cathéter intra-aortique dont la vessie distale est reliée à une chambre proche de l'autre extrémité du cathéter. Cette chambre est divisée en deux parties par une membrane. L'une des parties de cette chambre est reliée à une source de gaz sous pression destinée à remplir la vessie distale tandis que l'autre est reliée à une source de gaz sous pression destinée à modifier l'état de gonflement de la vessie par modification de la position de la membrane.

Ce cathéter nécessite deux sources de gaz sous pression. De plus, l'utilisation d'un gaz pour le gonflement de la vessie ne permet pas d'en contrôler le gonflement avec précision du fait de la compressibilité du gaz.

La présente invention tend à obvier à ces inconvénients et a pour objet un circuit pour cathéter devant être introduit dans un vaisseau sanguin présentant les caractéristiques énumérées à la revendication 1.

Dans ce cathéter, la mise sous pression interne de cette vessie ne se fait pas comme habituellement directement de l'extérieur du cathéter à partir d'une source d'alimentation mais au travers d'une seconde vessie pouvant jouer en quelque sorte et alternativement soit le rôle d'un réservoir émetteur soit le rôle d'un réservoir récepteur.

Le dessin annexé représente à simple titre d'exemple et non limitatif, une forme d'exécution dans son principe de l'objet de la présente invention.

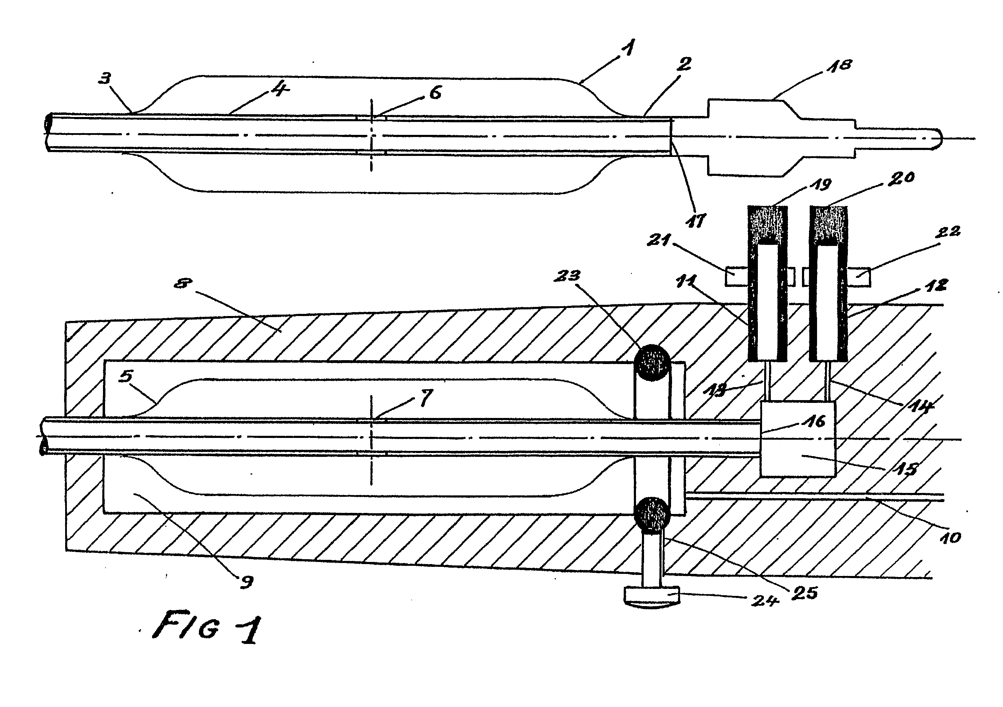

La figure 1 en est une vue générale en coupe et en deux parties.

Le circuit fermé pour le remplissage d'une vessie du cathéter représenté au dessin comprend une vessie 1 montée de façon étanche à ses deux extrémités 2 et 3 sur un tuyau 4, ainsi qu'un réservoir 5 également monté de façon étanche sur ce même tuyau 4 de la même façon que la vessie 1. Afin de permettre au circuit de remplir normalement sa fonction, il est nécessaire que ce tuyau 4 ou sa prolongation soit d'une part obstrué à ses deux extrémités et d'autre part possède un ou plusieurs orifices latéraux 6 et 7 débouchant respectivement à l'intérieur de la vessie 1 et du réservoir 5.

Le bâti 8 d'une poignée du cathéter non représentée, comprend une chambre 9 reliée par un canal 10 avec une source extérieure de fluide sous pression et peut ainsi être mise sous la pression d'un fluide. A la lecture du dessin figure 1, on distingue deux entrées bouchonnées 11 et 12 reliées par les deux canaux 13 et 14 et la chambre intermédiaire 15 permettent d'accéder au tuyau 4 par son extrémité 16, son autre extrémité 17 étant reliée à la partie du tuyau 4 portant la tête du cathéter représentée en 18 très schématiquement sur le dessin.

Dans une première opération, l'utilisateur de ce cathéter établira le vide maximum possible dans le circuit en perçant le bouchon 19 ou 20 de l'entrée 11 ou 12 par l'aiguille d'une seringue non représentée dont il retirera en arrière le piston en le maintenant dans cette position de recul. L'entrée utilisée est ensuite fermée par pincement au moyen du dispositif 21 ou 22 réservé à cet effet. Suite à la dépression ainsi obtenue, qui aura naturellement pour effet de resserrer la vessie 1 et le réservoir 5 autour du tuyau 4, l'utilisateur injectera par l'entrée restée libre 22 ou 21 au moyen d'une seconde seringue (non représentée préalablement remplie d'un fluide, en l'occurence d'un liquide, une quantité correspondant à la capacité du réservoir 5, généralement plus importante que la contenance de la vessie 1. On assure ainsi le remplissage de cette vessie 1, cette seconde entrée étant ensuite à son tour pincée au moyen de son dispositif correspondant 21 ou 22. A ce stade et pour que le contenu de la vessie 1 puisse retourner dans le réservoir 5 sous l'effet d'une compression quelconque, par exemple la contraction de la veine du patient, il est nécessaire de décompresser la chambre 9 par le canal 10 ou d'obtenir ce même résultat en déformant le O-Ring 23 au moyen du bouton 24 situé sur la tête de poignée est directement accessible par l'utilisateur.

On libère ainsi le canal 25 donnant sur l'extérieur du circuit fermé du présent cathéter.

Les avantages principaux de ce nouveau cathéter sont les suivants :

1. Il ne peut y avoir de surgonflage de la vessie, puisque celle-ci ne peut pas recevoir plus de liquide que la quantité qui est contenue dans le réservoir 5.

2. On ne peut appliquer de suppression aux parois du vaisseau sanguin du patient car le liquide contenu dans la vessie peut retourner dans le réservoir 5 lors de l'utilisation du cathéter lorsque la chambre 9 est reliée à l'échappement ou à l'air libre.

## Revendications

1. Circuit fermé pour cathéter comportant une vessie gonflable (1) située à l'extrémité distale d'un cathéter et reliée de façon étanche à un réservoir (5) situé à l'autre extrémité du cathéter, caractérisé par le fait que le réservoir est constitué par une seconde vessie gonflable; par le fait que cette seconde vessie gonflable est logée à l'intérieur d'une première chambre (9) pratiquée dans une poignée du cathéter; par le fait que ladite chambre est reliée d'une part à une source de fluide sous pression et d'autre part à l'extérieur de la poignée par un canal (25) muni de moyens d'obturation (24) et par le fait que l'intérieur de la seconde vessie (5) est relié à une seconde chambre (15) de la poignée, ladite seconde chambre étant reliée à l'extérieur de la poignée par l'intermédiaire de deux canaux (13, 14) munis de moyens d'obturation (19, 20).

2. Circuit selon la revendication 1, caractérisé par le fait que les moyens d'obturation du canal reliant ladite première chambre (9) de la poignée à l'extérieur sont constitués par un élément élastique (23) soumis à l'action d'un poussoir (24) de commande.

3. Circuit fermé pour cathéter selon les revendications 1 et 2, caractérisé par le fait que le circuit possède au moins une entrée destinée à sa mise sous dépression puis à son alimentation cette ou ces entrées étant munies chacune d'un dispositif (21, 22) pour assurer son ouverture ou sa fermeture.

4. Circuit fermé pour cathéter selon la revendication 3, caractérisé par le fait que la ou les entrées sont bouchées d'origine à leurs extrémités et donc prêtes à être piquées par une aiguille percée par exemple par l'aiguille d'une seringue médicale.

5. Circuit fermé pour cathéter selon la revendication 3, caractérisé par le fait que la ou les entrées sont ouvertes en se terminant vers l'extérieur par une sorte d'embouchure destinée à recevoir la partie en forme de téton cônique par exemple de la sortie d'une seringue médicale.

6. Circuit fermé pour cathéter selon la revendication 1, caractérisé par le fait que les vessies gonflables (1, 5) sont reliées entre elles par un tuyau (4) percé latéralement d'au moins un trou (6, 7) précisément à chaque endroit du tuyau recouvert par l'une dedites vessies.

**Claims**

1. Closed circuit for a catheter comprising an inflatable balloon (1) located at the distal end of a catheter and tightly connected to a tank (5) located at the other end of the catheter, characterized by the fact that the tank is formed by a second inflatable balloon; by the fact that this second inflatable balloon is located inside a first chamber (9) provided in a handle of a catheter; by the fact that the said chamber is connected on the one hand to a pressurizer fluid source and on the other hand to the outside of the handle by means of a channel (25) provided with closure means (24) and by the fact that the inside of the second balloon (5) is connected to a second chamber (15) of the handle, the said second chamber being connected to the outside of the handle by the intermediary of two channels (13, 14) provided with closure means (19, 20).

2. Circuit as claimed in claim 1, characterized by the fact that the closure means of the channel connecting the said first chamber (9) of the handle to the outside are formed by a resilient element (23) submitted to the action of a control push member (24).

3. Closed circuit for catheter according to claims 1 and 2, characterized by the fact that the circuit has at least one inlet intended to pressurize it and to feed it thereafter, this or these inlets being each provided with a device (21, 22) to permit its opening or its closure.

4. Closed circuit for catheter according to claim 3, caracterized by the fact that the inlet or the inlets are originally closed at their ends and ready to be pierced by needle for example the needle of a medical syringe.

5. Closed circuit for catheter according to claim 3, characterized by the fact that the inlet or the inlets are opened and ends outwardly by a kind of fitting intended to receive the conical stud shaped part for example the outlet at a medical syringe.

6. Closed circuit for catheter according to claim 1, characterized by the fact that the inflatable balloons (1, 5) are connected between themselves through a duct (4) laterally pierced with at least one hole (6, 7) precisely at each place of the duct where it is covered by said balloons.

**Patentansprüche**

1. Geschlossener Kreislauf für Katheter umfassend eine aufblasbare Blase (1), die am distalen Ende eines Katheters gelegen und dicht mit einem Behälter (5) verbunden ist, der am anderen Ende des Katheters gelegen ist, dadurch gekennzeichnet, daß der Behälter von einer zweiten aufblasbaren Blase gebildet ist; daß diese zweite aufblasbare Blase im Inneren einer ersten Kammer (9) positioniert ist, die in einem Handgriff des Katheters ausgebildet ist; daß die genannten Kammer einerseits mit einer Quelle für mit Druck beaufschlagtes Fluid und anderseits mit der Außenseite des Handgriffs durch einen Kanal (25) verbunden ist, der mit einer Verschlußeinrichtung (24) versehen ist, und daß das Innere der zweiten Blase (5) mit einer zweiten im Handgriff ausgebildeten Kammer (15) verbunden ist, und die genannte zweite Kammer mit der Außenseite des Handgriffs unter Zwischenschaltung von zwei Kanälen (13, 14) verbunden ist, die mit Verschlußeinrichtungen (19, 20) versehen sind.

2. Kreislauf nach Anspruch 1, dadurch gekennzeichnet, daß die Verschlußeinrichtungen des Kanals, der die genannte erste Kammer (9) des Handgriffs mit der Außenseite verbindet, von einem elastischen Element (13) gebildet werden, das der Wirkung eines Betätigungs-Druckknopfs (24) unterworfen ist.

3. Geschlossener Kreislauf für Katheter nach Anspruch 1 und 2, dadurch gekennzeichnet, daß der Kreislauf wenigstens einen Eingang aufweist, der für seine Beaufschlagung mit Unterdruck und dann für seine Speisung dient, wobei dieser Eingang bzw. diese Eingänge jeweils mit einer Vorrichtung (21, 22) versehen ist bzw. sind, die seine Öffnung oder sein Schließen gewährleistet.

4. Geschlossener Kreislauf für Katheter nach Anspruch 3, dadurch gekennzeichnet, daß der Eingang oder die Eingänge ursprünglich an seinen bzw. ihren Enden verschlossen bzw. abgedichtet sind und mit einer durchstoßenden Spitze bzw. Nadel aufgestochen werden können, z.B. der Nadel einer medizinischen Spritze.

5. Geschlossener Kreislauf für Katheter nach Anspruch 3, dadurch gekennzeichnet, daß der Eingang oder die Eingänge offen ist bzw. sind und nach außen hin in eine Art Mundstück bzw. Ansatz mündet bzw. münden, das bzw. der dazu bestimmt ist, den kegelstumpfförmigen Teil des Ausgangs z.B. einer medizinischen Spritze aufzunehmen.

6. Geschlossener Kreislauf für Katheter nach Anspruch 1, dadurch gekennzeichnet, daß die aufblasbaren Blasen (1, 5) untereinander mittels eines Rohres (4) verbunden sind, das seitlich von mindestens einem Loch (6, 7) genau an der jeweiligen Stelle durchbohrt ist, an der das Rohr von einer der genannten Blasen be- bzw. verdeckt ist.

FIG 1

EP 0 209 710 B1